# EUROPEAN PATENT SPECIFICATION

(11) **EP 3 416 747 B1**
(45) Date of publication and mention of the grant of the patent: **05.01.2022**
(21) Application number: 17702895.8
(22) Date of filing: 08.02.2017
(51) Int. Cl.: B05B 11/00, B65D 83/20, B65D 83/30, B65D 83/22

(54) **DISPENSER WITH ARTICULATED DISPENSING TUBE**
AUSTRAGEVORRICHTUNG MIT SCHWENKBAREM AUSGABEROHR
DISTRIBUTEUR AVEC TUBE DE DISTRIBUTION ARTICULÉE

(30) Priority: 16.02.2016 EP 16155950
(43) Date of publication of application: 26.12.2018
(73) Proprietor: Aziende Chimiche Riunite Angelini Francesco A.C.R.A.F. S.p.A., 00181 Roma (IT)
(72) Inventor: MASCIAMBRUNI, Roberto, 65124 Pescara (IT)
(74) Representative: Colombo, Stefano Paolo
(86) International application number: PCT/EP2017/052688
(87) International publication number: WO 2017/140542

(56) References cited:
- WO-A1-2008/088095
- WO-A1-2009/063518
- JP-A- 2002 355 307
- JP-U- H0 540 186
- US-A- 5 255 823

## Description

### BACKGROUND ART

The present invention relates, in general, to a spray dispenser-container assembly. In one embodiment, the present invention concerns a spray dispenser-container for a substance to be dispensed in a spray form in the oral cavity of a human being.

### STATE OF THE ART

There are known various substances, either pharmaceutical, para-pharmaceutical or homeopathic, used to soothe the sore throat. Some of these products are also used as antiseptic, anti-inflammatory and / or anti-pain in irritation of the mouth and gums, in gingivitis and stomatitis. In addition to such use, they may also be used before and / or after tooth extractions.

An example of such a product is marketed by the Applicant under the brand Tantum Verde Gola^{®}. This product contains a nonsteroidal anti-inflammatory and an antiseptic. There are several ways to use that product. According to one way to use such product, the target zone (for example, the oral cavity or the pharynx) is approached through a cannula and a certain amount of product is sprayed towards the target zone.

A container which is presently used for the administration according to the above spray mode includes a substantially cylindrical vial closed on top by a metal ring that blocks a pump. The pump is hand operated by the user pressing with a finger on top of a button. As a result of the drive of the pump, a predetermined amount of the substance is sucked from a straw within the vial and dispensed towards an outlet nozzle. The outlet nozzle, in use, communicates with a longitudinal cavity of a cannula which is configured to atomize the predetermined amount of substance.

In the known container, advantageously, the cannula can take a rest (or not in use) configuration and a configuration of use (mentioned above). In the rest configuration, the axis of the cannula is substantially parallel to the axis of the vial and is located next to the outer wall of the vial. In the configuration of use, the axis of the cannula is located substantially perpendicular to the axis of the vial. The passage from one configuration to the other is allowed for the fact that the cannula is hinged to the nozzle.

The known container described above is rather efficient and practical. However, the Applicant has set itself the aim of improving it.

Each of JP H05 40186 U and JP 2002 355307 A discloses a sprayer for a throat.

WO 2008/088095 A1 discloses a pumping device with collapsible nozzle.

WO 2009/063518 A1 discloses a dispensing head for dispensers of fluid products.

### SUMMARY OF THE INVENTION

First, the Applicant has noticed that the cannula, when in the rest or non-use position, is completely exposed to the surrounding environment. Disadvantageously, it may be contaminated by contact with other objects or can bend and, in some cases, even break.

The risk of the cannula to bend or break leads to endow the vial with a rather rigid cannula.

The Applicant has set itself the aim of providing a container of the above type configured so as to protect the full length of the cannula in the configuration of non-use. According to the present invention, the container is configured so as to have a cannula protection housing configured to protect all the cannula when it is in the non-use configuration. If the cannula is not fully protected, it might become damaged or dirty. If a portion of the cannula is not protected, it may become entangled if it is inserted in apocket or the like.

In addition to the above aim of providing a container of the above type configured so as to protect the cannula in the configuration of non-use, the Applicant has also set itself the aim of providing a container preventing any inadvertent rotation of the button and/or of the pump during use. As it is known, typical pumps and corresponding buttons for dispensing a substance from a container provide a dispensing position and a blocked position. In the blocked position the dispensing of the substance is prevented. However, disadvantageously, the blocked position is not identified. This means that it might happen that when the user presses the button, the pump is in the block position and it is unable to dispense the substance.

This situation is highly uncomfortable for a user who is going to spray a substance in his/her mouth or throat. The user needs to move the dispenser away and to find the correct dispensing position. Again, as this position is not identified, the user has two options.

According to the first option, he/she turns the button (with the cannula in its extended configuration) by some degrees and hopes to have found the dispensing position. Then he/she inserts again the cannula in in his/her mouth or throat and presses the button. However, the performed rotation could reveal itself inappropriate and the pump could still be in the blocked position.

According to the second option, the user makes "dummy" sprays in order to identify the correct dispensing position while the cannula is not in the mouth or throat. This has the disadvantage that a lot of substance is wasted.

While the problem of directionality is a problem which is felt by users in general, it is highly felt by blind users or the like. For those people, having a unique predetermined position for grasping the bottle and directing the cannula to the target area is extremely important.

The Applicant has also noted that in the known spray containers, the button can perform only a limited movement under the pressure exercised by a user's finger. This means that only a limited amount of substance can be sprayed. It would be desirable to provide a spray container able to spray a larger amount of substance for single spray action.

According to the present invention, at least a portion of the above objects is reached by a dispenser-container assembly comprising, *inter alia,* a dispensing cannula configured to assume a rest configuration in a cannula housing and wherein the dispensing button can only perform a translational movement substantially without rotation. In this manner, the pump can not assume the blocked position wherein spraying is prevented. The user will not run the risk to be unable to spray when he/she is spraying a substance in his/her mouth or throat.

In some embodiments, the spraying is prevented with the cannula in its rest configuration.

According to the present invention, there is provided a dispenser-container assembly for containing a substance and for delivering such a substance in a spray (or atomized) form in a target area, the assembly comprising:
a container and a pump associated to said container to suck an amount of substance from said container and deliver it towards an outlet nozzle;
a button to actuate said pump, said button comprising a button lateral surface;
a body associable to the container and enclosing at least a portion of said button lateral surface;
a dispensing cannula configured to assume a rest configuration and a configuration of use; and
a cannula housing within which substantially the full length of the cannula can be held in a secured way when the assembly is in its rest configuration,
wherein said body is configured to retain and guide said button in an essentially translational movement,
wherein the body comprises a member which projects upwardly from said container, wherein an inner surface of said member directly faces a corresponding portion of the button lateral surface,
wherein the button has a non-circular shape identified by a cross-section with a minor axis and a major axis of a length greater than the length of said minor axis.

In the present description and claims, the expression "essentially translational movement" is used for indicating that the button performs only a translational movement without any rotational movement except a minimal rotational movement, for instance a rotational movement caused by plays between components. Therefore, in principle the above expression provides for a translational movement with a rotational movement of very few degrees (1° to 5°, preferably 1° to 3°, more preferably 1° to 2°).

In embodiments, the member follows at least a part of the contour of the container.

In embodiments, the member comprises two wings which extend upwards following a part of the contour of the container.

In embodiments, the two wings are separated by a slit for the cannula.

In embodiments, the cannula housing comprises a channel which extends substantially along an axis parallel to a longitudinal axis of the assembly.

In embodiments, there is provided a gripping notch configured to grasp the cannula with the fingertips or fingernails and bring, at least partially, the cannula outside of the housing.

In embodiments, the channel is closed at the bottom so that actuation of said pump is prevented when the dispensing cannula assumes the rest configuration.

In embodiments, the cannula is rotatably hinged to the button.

In embodiments, the assembly has a non-circular shape identified by a cross-section with a minor axis and a major axis of a length greater than the length of said minor axis.

In embodiments, the assembly comprises a curved face adapted to come into contact with the palm of a user's hand and a flat face adapted to come into contact with the fingers of the user's hand.

The substance can be a mouthwash or similar to be dispensed in a spray form in the oral cavity of a human being.

### BRIEF DESCRIPTION OF FIGURES

The present invention will become clearer from the following description, provided by way of example and not limitation, to be read with reference to the accompanying drawings, in which,
- Figure 1 shows a known dispenser-container assembly;
- Figure 2 shows a dispenser-container assembly according to the present invention;
- Figure 3 shows a dispenser-container assembly according to the present invention without the pump button;
- Figure 4A is a side view of the dispensing container according to the present invention;
- Figure 4B is a cross-section taken along line B-B of Figure 4;
- Figure 5 is a front view of the dispenser-container assembly according to the present invention;
- Figure 5A is a cross-section taken along line A-A of Figure 5; and
- Figure 6 is a top view of the dispenser-container assembly according to the present invention.

### DETAILED DESCRIPTION

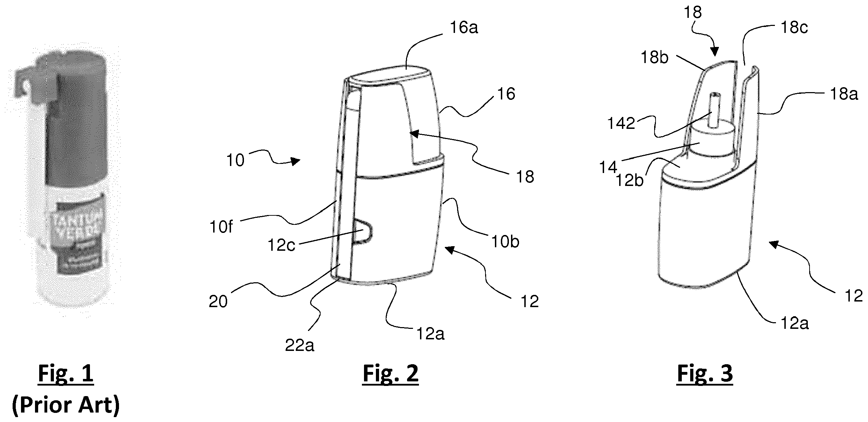

Figure 1 shows a known dispenser-container in its rest configuration (i.e. in the configuration of not use).

The container comprises a substantially cylindrical vial which is closed on top by a ring nut (not shown) through which a pump is mounted. The pump is manually actuated by the user by pressing on the top. As a result of operating the pump, a predetermined amount of the substance is sucked from a straw within the vial and dispensed towards an outlet nozzle, typically arranged in axis with the vial. The outlet nozzle communicates with a channel section which is connected to the operation button and, in the configuration of use, communicates with a rigid cannula whose end is configured to atomize the sucked predetermined amount of substance.

In the rest configuration, the axis of the cannula is substantially parallel to the axis of the vial and is located next to the outer wall of the vial. In the configuration of use (not shown), the axis of the cannula is located substantially perpendicular to the axis of the vial. The passage from one configuration is made for the fact that the cannula is hinged to the button.

Figure 2 is a schematic axonometric view of the dispenser-container assembly 10 according to one embodiment of the present invention. Figure 3 is also an axonometric view of the assembly 10 of Figure 2 but taken from a different angle and with the button removed.

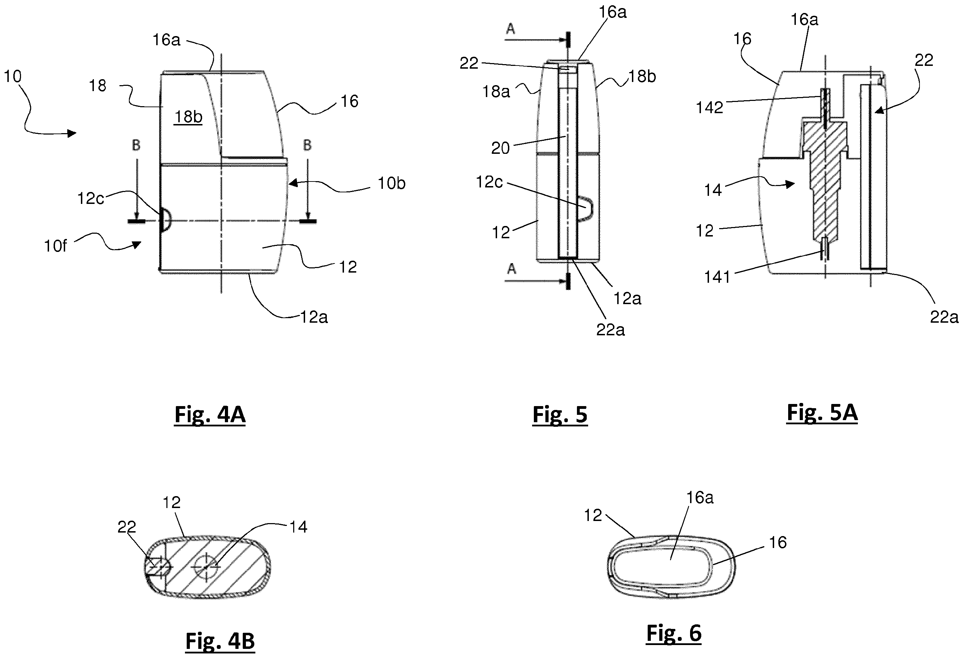

The assembly 10 comprises a container 12 for containing the substance to be dispensed. Preferably, the container 12 has a section roughly rounded rectangular (Figure 4B). In other embodiments, it could have an elliptical cross section, oval or the like. In any case, it is preferable that the cross section of the container 12 is not circular ("acircular") with a major axis and a minor axis of different length. Preferably, the major axis is about twice the minor axis.

The shape of the assembly is also advantageous in that it allows to apply a rather large label on a generally planar surface. This is in contrast to a cylindrical surface with circular cross-section. For such a bottle, the label should be placed on the circular wall of the container and this is unconvenient for a user that has to read it. A more planar label is more readable and it is also advantageous for writing information in Braille or for any of a bar code, a QR code or the like.

Furthermore, the cross-section of the assembly of the present invention allows to store it in a breast pocket or the like. It will remain more stable in such a pocket and will project in a limited amount with respect to circular cross-sections.

Preferably, the section of the container 12 in the vicinity of its lower base 12a is lower and increases gently towards the upper base.

Preferably, as clearly visible in Figure 4, the front side 10f of the assembly 10 (and thus also of the container 12) is substantially flat and is substantially perpendicular to the base 12a.

Preferably, still referring to Figure 4, the back side 10b of the assembly 10 is substantially curved and forms an arc of a circle or the like. This shape is considered advantageous from the viewpoint of improving handling and ergonomics, as will become clear later.

In alternative embodiments, the front side 10f and back side 10b could both be substantially planar and perpendicular to the base or both curved arc of a circle.

The container 12 may be in any material, but is preferably in a thermoplastic material such as polyethylene, polyethylene terephthalate or the like and is obtained by molding.

The lower base 12a of the container is preferably flat. Preferably, the upper base 12b of the container ends with a screw neck for coupling to the container 12, a pump body 14, shown schematically in Figure 5A. The pump body 14 is of a known type: comprises a suction pipe 141 open at the bottom and an actuating rod 142 connected to actuating button 16.

The dispensing container assembly 10 of the present invention preferably comprises a body 18 associable to the container 12 to provide an abutment and retaining means for the trigger button 16 both when the button 16 is pressed and when the button 16 is not urged. Preferably, the body 18 includes a member comprising two wings 18a and 18b which extend upwards. Preferably, the member follows a part of the contour of the container 12. View from the front, the two wings 18a, 18b are preferably separated by a slit for the cannula 20 which will be described later in this description. It should be remarked that thanks to the above shape, the cannula 20 is fully protected along its full length. In its upper part, the dispenser-container assembly 10 comprises a pump trigger button 16. The button 16 is configured to cooperate with actuating rod 142 of the pump 14 and to perform a translation movement in the direction of the axis of the assembly 10. Preferably, the button 16 is retained by the two wings 18a, 18b of the body 18. Preferably, the button 16 is shaped substantially symmetrically with respect to the container 10.

In other embodiments, not shown, the essentially translational movement of the button can be obtained by providing a guide arrangement. Such guide arrangement can be made, for example, with a groove which extends longitudinally and which is made in the button (or in the body 18) and with a mating longitudinal projection in the body 18 (or in the button).

The body 18 and the container 12 are connected together in a stable manner. According to one embodiment, there are provided undercuts and corresponding engagements in the body 18 and/or in the container 12 members to provide said stable connection.

In other embodiments, the body 18 and the container 12 can be connected through gluing or welding, for example ultrasonic welding. In other embodiments, the body 18 and the container 12 are mutually connected through the threaded neck of the container 12.

The rod 142 of the pump 14 is preferably connected to a flexible tube inserted into a rigid cannula and which ends with one end configured to atomize a predetermined amount of the substance sucked through the pump.

According to the present invention, the assembly 10 comprises a housing 22 within which the cannula 20 can be safely held when the assembly is in its rest configuration or non-use configuration. Preferably, the housing 22 is in the form of a channel which extends substantially along an axis parallel to the longitudinal axis of the assembly 10. The channel 22 is formed in the button 16 as regards the upper part of the assembly 10 and into the container 12 as regards its lower part.

The channel 22 preferably has a greater depth in correspondence with the button 16 and a smaller depth along the height of the container 12.

Preferably, the channel 22 is closed (22a) at the base of the container 12a. In this way, the end of the cannula 20, configured to atomize, remains completely protected. Moreover, advantageously, this closure serves as a block and prevents the button 16 is pressed. Therefore, escaping of substance drawn by the pump 14 when the assembly 10 is in its rest position is prevented.

Preferably, the container also includes a gripping notch 12c which is provided for grasping the cannula 20 with the tip of the fingers, or the nails, of a hand and bring the cannula 20 outside of the housing 22. In other words, thanks to the gripping notch 12c, the cannula 20 can be rotated a few degrees and then, when at least the end of the cannula 20 is out of the housing 22, the rotation of the cannula is completed until the configuration of use (in which the cannula 20 is substantially perpendicular to the longitudinal axis of the assembly 10) is reached.

After use, the cannula 20 is moved back into the housing channel 22 by rotating the cannula downwardly.

As mentioned above, the container-dispenser according to the present invention is particularly practical, easy to handle and ergonomic.

First, the thickness (minor axis of the cross section) is less than the width (major axis of the cross section). This allows to grasp it firmly even by users with small hands, for example children or women. The user can grip the assembly 10 in such a way that the rear face (circular arc-shaped or otherwise curved) may be received in the palm of his hand and the front face (flat) is in contact with the phalanges of the fingers of the user's hand. In this way, the gripping is convenient and intuitive for the user.

The user can exert greater pressure force on the button because it uses less force to keep the assembly between the palm and fingers.

The upper surface of the button is broad. This allows it to be pressed smoothly and efficiently even by people with a large thumb.

Also, another advantage of the present invention is given by a more precise directionality. Once again, thanks to the tapered shape (or in any case to the shape which is not circular), the user is led to grip and retain the assembly in the sole and appropriate manner. In this way, the user does not run the risk that the cannula is incorrectly oriented. It should be noted in fact that when the cannula is placed in the mouth, the user does not see it. A user of a known dispenser is unable to understand how it is oriented. The greater certainty of the guidance of the cannula leads naturally to reach more precisely the target area within the oral cavity.

In any case, the main advantage of the present invention is constituted by the possibility to keep the whole cannula repaired (and in particular its end) when the assembly is not in use. This guarantees a better hygiene and avoids the risk of contamination. Moreover, it is virtually annulled the risk that the cannula from bending or breaking. It is also substantially canceled the risk that the tip from getting clogged by the presence of dust or hair.

Furthermore, the closed end of the channel 22 functions as a block and prevents the accidental activation of the pump when the assembly is in its configuration of non-use.

According to one embodiment, the container of the assembly is configured to hold about 15 ml of substance. According to another embodiment, the container of the assembly is configured to hold about 30 ml of substance. This substance may be a mouthwash or the like.

According to one embodiment of the invention, the container may have a front surface of about 20 mm and a side surface of about 40 mm. The overall height of the assembly can be about 60-65 mm and the housing channel of the cannula can have a length of about 60 mm.

## Claims

1. A dispenser-container assembly (10) for containing a substance and delivering it in a spray form in a target area, the assembly (10) comprising:
a container (12) and a pump (14) associated to said container (12) to suck an amount of substance from said container (12) and deliver it towards an outlet nozzle (142);
a button (16) to actuate said pump (14), said button (16) comprising a button lateral surface (16b);
a body (18) associable to the container (12) and enclosing at least a portion of said button lateral surface (16b); and
a dispensing cannula (20) configured to assume a rest configuration and a configuration of use;
**characterized in that**
the assembly (10) further comprises a cannula housing (22) within which substantially the full length of the cannula (20) can be held in a secured way when the assembly (10) is in its rest configuration, and **in that**
said body (18) is configured to retain and guide said button (16) in an essentially translational movement,
said body (18) comprises a member which projects upwardly from said container (12), wherein an inner surface of said member directly faces a corresponding portion of the button lateral surface (16b), and
said button (16) has a non-circular shape identified by a cross-section with a minor axis and a major axis of a length greater than the length of said minor axis.

2. The assembly (10) of claim 1, wherein said member follows at least a part of the contour of the container (12).

3. The assembly (10) of claims 1 or 2, wherein said member comprises two wings (18a, 18b) which extend upwards following a part of the contour of the container (12).

4. The assembly (10) of claim 3, wherein said two wings (18a, 18b) are separated by a slit for the cannula (20).

5. The assembly (10) of any of preceding claims, wherein said cannula housing (22) comprises a channel which extends substantially along an axis parallel to a longitudinal axis of the assembly (10).

6. The assembly (10) of any of preceding claims, also comprising a gripping notch (12c) configured to grasp the cannula (20) with the fingertips or fingernails and bring, at least partially, the cannula (20) outside of the housing (22).

7. The assembly (10) of claim 5, wherein said channel (22) is closed at the bottom (22a) so that actuation of said pump (14) is prevented when the dispensing cannula (20) assumes said rest configuration.

8. The assembly (10) of any one of the preceding claims, wherein said cannula (20) is hinged rotatably to said button (16).

9. The assembly (10) of any one of the preceding claims, wherein said assembly (10) has a non-circular shape identified by a cross-section with a minor axis and a major axis of a length greater than the length of said minor axis.

10. The assembly (10) of claim 9, comprising a curved face (10b) adapted to come into contact with the palm of a user's hand and a flat face (10f) adapted to come into contact with the fingers of the user's hand.

11. The assembly (10) of any one of the preceding claims, wherein said substance is a mouthwash or similar to be dispensed in a spray form in the oral cavity of a human being.

## Patentansprüche

1. Spender-Behälteranordnung (10) zur Aufnahme einer Substanz und deren Abgabe in Form eines Sprays in ein Zielgebiet, wobei die Anordnung (10) umfasst:
- einen Behälter (12) und eine dem Behälter (12) zugeordnete Pumpe (14), um eine Substanzmenge aus dem Behälter (12) anzusaugen und zu einer Auslassdüse (142) hin abzugeben;
- einen Knopf (16) zum Betätigen der Pumpe (14), wobei der Knopf (16) eine Knopfseitenfläche (16b) umfasst;
- einen Körper (18), der mit dem Behälter (12) verbindbar ist und wenigstens einen Teil der Knopfseitenfläche (16b) umschließt; und
- eine Spenderkanüle (20), die so ausgelegt ist, dass sie eine Ruhekonfiguration und eine Gebrauchskonfiguration einnehmen kann;
**dadurch gekennzeichnet, dass**
- die Anordnung (10) außerdem ein Kanülengehäuse (22) umfasst, in welchem im Wesentlichen die gesamte Länge der Kanüle (20) in einer gesicherten Weise aufgenommen werden kann, wenn sich die Anordnung (10) in ihrer Ruhekonfiguration befindet, und dadurch, dass
- der Körper (18) eingerichtet ist, den Knopf (16) in einer im Wesentlichen translatorischen Bewegung zu halten und zu führen,
- wobei der Körper (18) ein Bauteil umfasst, welches von dem Behälter (12) nach oben vorsteht, wobei eine innere Fläche des Bauteils einem entsprechenden Abschnitt der Knopfseitenfläche (16b) direkt gegenüberliegt, und
- der Knopf (16) eine nicht-kreisförmige Form aufweist, die durch einen Querschnitt mit einer Nebenachse und einer Hauptachse mit einer größeren Länge als der Länge der Nebenachse bestimmt wird.

2. Anordnung (10) gemäß Anspruch 1, wobei das Bauteil wenigstens einem Teil der Kontur des Behälters (12) folgt.

3. Anordnung (10) gemäß Anspruch 1 oder 2, wobei das Bauteil zwei Flügel (18a, 18b) umfasst, die sich nach oben erstrecken und einem Teil der Kontur des Behälters (12) folgen.

4. Anordnung (10) gemäß Anspruch 3, wobei die beiden Flügel (18a, 18b) durch einen Schlitz von der Kanüle (20) getrennt sind.

5. Anordnung (10) gemäß einem der vorhergehenden Ansprüche, wobei das Kanülengehäuse (22) einen Kanal umfasst, der sich im Wesentlichen entlang einer Achse erstreckt, die parallel zu einer Längsachse der Anordnung (10) ist.

6. Anordnung (10) gemäß einem der vorhergehenden Ansprüche, die außerdem eine Greifkerbe (12c) umfasst, die so ausgelegt ist, dass die Kanüle (20) mit den Fingerspitzen oder Fingernägeln gegriffen werden und die Kanüle (20) wenigstens teilweise aus dem Gehäuse (22) herausgeführt werden kann.

7. Anordnung (10) gemäß Anspruch 5, wobei der Kanal (22) am Boden (22a) geschlossen ist, so dass eine Betätigung der Pumpe 814) verhindert wird, wenn die Spenderkanüle (20) die Ruhekonfiguration einnimmt.

8. Anordnung (10) gemäß einem der vorhergehenden Ansprüche, wobei die Kanüle (20) drehbar an dem Knopf (16) angelenkt ist.

9. Anordnung (10) gemäß einem der vorhergehenden Ansprüche, wobei die Anordnung (10) eine nicht-kreisförmige Form aufweist, die durch einen Querschnitt mit einer Nebenachse und einer Hauptachse mit größerer Länge als der Länge der Nebenachse bestimmt ist.

10. Anordnung (10) gemäß Anspruch 9, umfassend eine gekrümmte Fläche (10b), die ausgelegt ist, mit der Handfläche eines Benutzers in Kontakt zu kommen, und eine ebene Fläche (10f), die ausgelegt ist, mit den Fingern der Hand des Nutzers in Kontakt zu kommen.

11. Anordnung (10) gemäß einem der vorhergehenden Ansprüche, wobei die Substanz eine Mundspülung oder etwas Ähnliches ist, die in Form eines Sprays in die Mundhöhle eines Menschen abgegeben werden kann.

## Revendications

1. Ensemble récipient - distributeur (10) destiné à contenir une substance et la distribuer sous forme de pulvérisation dans une zone cible, l'ensemble (10) comprenant :
un récipient (12) et une pompe (14) associée audit récipient (12) pour aspirer une quantité de substance dudit récipient (12) et la distribuer vers une buse de sortie (142) ;
un bouton (16) pour actionner ladite pompe (14), ledit bouton (16) comprenant une surface latérale de bouton (16b) ;
un corps (18) pouvant être associé au récipient (12) et enfermant au moins une partie de ladite surface latérale de bouton (16b) ; et
une canule de distribution (20) configurée pour adopter une configuration de repos et une configuration d'utilisation ;
**caractérisé en ce que** :
l'ensemble (10) comprend en outre un logement de canule (22) à l'intérieur duquel sensiblement toute la longueur de la canule (20) peut être contenue d'une manière sécurisée lorsque l'ensemble (10) est dans sa configuration de repos, et **en ce que** :
ledit corps (18) est configuré pour retenir et guider ledit bouton (16) dans un mouvement essentiellement de translation,
ledit corps (18) comprend un élément qui fait saillie vers le haut à partir dudit récipient (12), dans lequel une surface interne dudit élément fait directement face à une partie correspondante de la surface latérale de bouton (16b), et
ledit bouton (16) a une forme non circulaire identifiée par une section transversale avec un axe mineur et un axe majeur d'une longueur supérieure à la longueur dudit axe mineur.

2. Ensemble (10) selon la revendication 1, dans lequel ledit élément suit au moins une partie du contour du récipient (12).

3. Ensemble (10) selon les revendications 1 ou 2, dans lequel ledit élément comprend deux ailes (18a, 18b) qui s'étendent vers le haut en suivant une partie du contour du récipient (12).

4. Ensemble (10) selon la revendication 3, dans lequel lesdites deux ailes (18a, 18b) sont séparées par une fente pour la canule (20).

5. Ensemble (10) selon l'une quelconque des revendications précédentes, dans lequel ledit logement de canule (22) comprend un canal qui s'étend sensiblement le long d'un axe parallèle à un axe longitudinal de l'ensemble (10).

6. Ensemble (10) selon l'une quelconque des revendications précédentes, comprenant également une encoche de préhension (12c) configurée pour saisir la canule (20) avec le bout des doigts ou les ongles et amener, au moins partiellement, la canule (20) à l'extérieur du logement (22).

7. Ensemble (10) selon la revendication 5, dans lequel ledit canal (22) est fermé au niveau du fond (22a) de sorte que l'actionnement de ladite pompe (14) est empêché lorsque la canule de distribution (20) adopte ladite configuration de repos.

8. Ensemble (10) selon l'une quelconque des revendications précédentes, dans lequel ladite canule (20) est articulée, de manière rotative, par rapport audit bouton (16).

9. Ensemble (10) selon l'une quelconque des revendications précédentes, dans lequel ledit ensemble (10) a une forme non circulaire identifiée par une section transversale avec un axe mineur et un axe majeur d'une longueur supérieure à la longueur dudit axe mineur.

10. Ensemble (10) selon la revendication 9, comprenant une face incurvée (10b) adaptée pour venir en contact avec la paume de la main d'un utilisateur et une face plate (10f) adaptée pour venir en contact avec les doigts de la main de l'utilisateur.

11. Ensemble (10) selon l'une quelconque des revendications précédentes, dans lequel ladite substance est un bain de bouche ou similaire destiné à être distribué sous forme de pulvérisation dans la cavité buccale d'un être humain.
